# EUROPÄISCHE PATENTANMELDUNG

(11) **EP 4 779 648 A1**
(43) Veröffentlichungstag der Anmeldung: **22.07.2026**
(21) Anmeldenummer: 25152132.4
(22) Anmeldetag: 15.01.2025
(51) Int. Cl.: G16H 30/40, G16H 50/20, G16H 20/10, A61K 39/35

(54) **VERFAHREN ZUR HERSTELLUNG VON PERSONALISIERTEN UND INDIVIDUELLEN ALLERGEN-ZUSAMMENSETZUNGEN**

(71) Anmelder: Allergo AI Diagnostic & Therapeutics Deutschland Inc. & Co KG, 19258 Boizenburg (DE)
(72) Erfinder: Schönfelder, Daniel, 19258 Boizenburg (DE)
(74) Vertreter: Simandi, Claus

(57) **Zusammenfassung**

Gegenstand der vorliegenden Erfindung ist ein Verfahren zur Herstellung von pharmazeutischen Zusammensetzungen enthaltend Allergene zur Verwendung in der Immuntherapie oder in der Prophylaxe oder Behandlung von Allergien, Nahrungsmittelunverträglichkeit oder Arzneimittelunverträglichkeit.

## Beschreibung

Gegenstand der vorliegenden Erfindung ist ein Verfahren zur Herstellung von pharmazeutischen Zusammensetzungen enthaltend Allergene zur Verwendung in der Immuntherapie oder in der Prophylaxe oder Behandlung von Allergien, Nahrungsmittelunverträglichkeit oder Arzneimittelunverträglichkeit.

In der Allergologie werden verschiedene Hauttests auch als diagnostischen Verfahren eingesetzt, die sich in ihrer Art und Technik nach dem zu beurteilenden klinischen Bild und dem potenziell ursächlichen Allergen richten, darunter der Prick-Test, Patch-Test und intradermale Reaktionen.

Die Gründe für den weit verbreiteten Einsatz von Prick-Testen liegen vor allem in den folgenden Vorteilen, die ein solcher Test bietet: hohe Effizienz oder Genauigkeit; Einfachheit der Durchführung und Auswertung; minimale Invasivität, sodass insbesondere Kinder eine hohe Akzeptanz aufweisen; nahezu kein Risiko von Nebenwirkungen; geringe Kosten auch für das verwendete Material (Allergene, Stech- und Lesegeräte).

Beim Prick-Test wird geprüft, ob das Immunsystem des Patienten allergisch auf bestimmte Substanzen - also Allergene - reagiert. Dies geschieht durch das gezielte Auftragen von Allergenlösungen auf die Haut, meist an der Innenseite des Unterarms, und ein leichtes Anritzen der Haut, um die Aufnahme (Penetration) des Allergens zu ermöglichen.

Prick-Teste können bei Probanden eine unmittelbare Hautreaktion auslösen, wobei ein Allergen in die Haut penetriert, aktiviert das Immunsystem sogenannte Mastzellen in der Haut. Diese setzen Histamin frei, was zu i.) einer Erweiterung der Blutgefäße mit sichtbarer Rötung (Erythem), und ii.) eine Erhöhung der Durchlässigkeit der Gefäße zu einer sichtbaren Schwellung bzw. Bildung von Quaddeln führt.

In einem ersten Schritt erfolgt das Auftragen der Allergenlösungen in definierten Abständen auf die Haut. Zusätzlich werden Kontrolllösungen aufgetragen: Positive Kontrolle (Histamin) löst eine Hautreaktion aus. Negative Kontrolle (Kochsalzlösung) löst keine Reaktion aus.

Mit einer feinen Lanzette oder Nadel wird die Haut durch die aufgetragene Allergenlösung hindurch oberflächlich angeritzt.

Alternativ kann auch ein vorbereiteter Mikronadelarray mit aufgetragenen Allergenlösungen verwendet werden.

Es wird 15 bis 20 Minuten gewartet, um die Reaktionen der Haut zu beobachten.

Es wird geprüft, ob an den Stellen der Allergenlösungen sichtbare Reaktionen auftreten, nämlich:
a.) Rötung: Ein Anzeichen für eine Immunreaktion,
b.) Quaddel: Eine Schwellung bzw. Bildung einer Quaddel, die zeigt, dass das Immunsystem sensibilisiert ist.

Die Größe und Intensität der sichtbaren Reaktionen werden gemessen und mit den Kontrollpunkten verglichen, wie z.B. in US2017262985A1 beschrieben.

Eine größere Quaddel bei einem Allergen als bei der positiven Kontrolle deutet auf eine allergische Sensibilisierung hin.

Der Begriff "allergische Sensibilisierung" beschreibt den Zustand, in dem das Immunsystem eines Säugetieres insbesondere Mensch auf eine normalerweise harmlose Substanz, wie Pollen, Hausstaubmilben oder bestimmte Nahrungsmittel oder Arzneimittel, überempfindlich reagiert. Diese Substanz wird erfindungsgemäß als Allergen bezeichnet. Bei einer allergischen Sensibilisierung hat der Körper spezifische Antikörper, insbesondere Immunglobuline, wie IgE gegen das Allergen gebildet, obwohl noch keine sichtbaren allergischen Symptome auftreten müssen. Eine Sensibilisierung bedeutet, dass das Immunsystem auf ein Allergen vorbereitet ist. Symptome einer allergischen Reaktion treten jedoch erst auf, wenn das sensibilisierte Immunsystem tatsächlich auf das Allergen reagiert.

Bei Allergien wird zur Behandlung die Desensibilisierung oder spezifische Immuntherapie (SIT), auch als Hyposensibilisierung bezeichnet, durchgeführt. Ziel ist es, das Immunsystem an die allergieauslösenden Stoffe (Allergene) zu gewöhnen, sodass das Immunsystem weniger stark oder gar nicht mehr darauf reagiert.

Die Desensibilisierung erfolgt in der Regel mit Allergenextrakten, also speziell aufbereitete Lösungen oder Injektionen, die das Allergen in gereinigter Form enthalten, solche wie Pollen (Gräser, Bäume), Hausstaubmilben, Tierhaare, Schimmelpilze, Insektengifte (z. B**.** von Bienen oder Wespen).

Folgende Applikationsformen sind beschrieben:
Subkutane Immuntherapie (SCIT): Das Allergen wird in ansteigender Konzentration unter die Haut gespritzt.
Sublinguale Immuntherapie (SLIT): Das Allergen wird in Form von Tropfen oder Tabletten unter die Zunge gegeben.

Orale Immuntherapie (OIT): Vor allem bei Nahrungsmittelallergien wird das Allergen in sehr kleinen, kontrollierten Mengen über den Mund aufgenommen.

Die Immuntherapie beginnt mit einer Einschleichphase, in der die Dosis langsam gesteigert wird. Danach folgt die Erhaltungsphase, in der eine konstante Dosis regelmäßig verabreicht wird. Die Therapie dauert in der Regel 3 bis 5 Jahre.

Allerdings ist die Desensibilisierung zumeist nicht hinreichend zielführend und erfolgreich, da der individuelle Schweregrad einer Allergie und bestehende Sensibilisierung in der Therapie nicht berücksichtigt wird.

Auch andere Faktoren bleiben unberücksichtigt, insbesondere solche wie Körpergewicht, Größe, Alter, Geschlecht, IgE-Level im Blut, Body Mass Index (BMI), saisonale Allergenbelastung, Vorerkrankungen.

Gegenstand der Erfindung ist daher ein Verfahren zur Herstellung von pharmazeutischen Zusammensetzungen enthaltend Allergene aufweisend folgende Schritte:
i.) Bildliche Aufnahme von mindestens zwei oder mehr Quaddeln, welche durch einen Prick-Test erzeugt sind, wobei jede Quaddel mindestens ein verschiedenes Allergen repräsentiert,
ii.) Bestimmung von mindestens einem Parameter ausgewählt aus der Gruppe Größe oder Volumen aus der bildlichen Aufnahme von mindestens zwei oder mehr Quaddeln aus i.),
iii.) Bestimmung der Verhältnisse der Parameter aus ii.) von mindestens zwei oder mehr Quaddeln,
dadurch gekennzeichnet,
dass die pharmazeutische Zusammensetzung entsprechend dem Verhältnis aus iii.) Allergene enthält.

Daher betrifft die Erfindung in einem weiteren Schritt ein Verfahren zur Herstellung von pharmazeutischen Zusammensetzungen enthaltend Allergene:
iv.) wobei das Verhältnis der Allergene aus iii.) durch die Mengen an Allergenen in Gew. % repräsentiert ist, und die Mengen nach mindestens einem Parameter bestimmt werden ausgewählt aus der Gruppe Körpergewicht, Größe, Alter oder Geschlecht eines Patienten.

Gemäß Schritt iv.) kann vorteilhaft die Dosis für einen Probanden individuell eingestellt werden.

Daher betrifft die Erfindung in einem weiteren Schritt ein Verfahren zur Herstellung von pharmazeutischen Zusammensetzungen enthaltend Allergene:
v.) wobei das Verhältnis der Allergene aus iii.) durch die Mengen an Allergenen in Gew. % repräsentiert ist, und die Mengen nach mindestens einem Parameter bestimmt werden ausgewählt aus der Gruppe IgE-Level im Blut, Body Mass Index (BMI), saisonale Allergenbelastung, Vorerkrankungen.

Gemäß Schritt v.) kann vorteilhaft die Dosis für einen Probanden individuell eingestellt werden.

Beispielsweise beträgt das Verhältnis von 2 bestimmten Quaddeln gemäß iii.) 1 : 1, so sind die jeweiligen Allergene zu gleichen Gew. % in einer pharmazeutischen Zusammensetzung enthalten.

Die bildliche Aufnahme gemäß
i.) kann beispielsweise mit einer Kamera oder Videogerät erfolgen. Insbesondere ist die Kamera eines handelsüblichen Smartphones ausreichend. Bevorzugt ist die Aufnahme eines hochauflösenden Bildes der Quaddel.

In einer weiteren bevorzugten Ausführungsform werden ein oder mehrere ARUCO-Marker verwendet. Ein Aruco-Marker ist ein quadratischer, schwarz-weißer Marker, der erfindungsgemäß für die genaue Bestimmung von Kameraparametern bzw. Kalibrierung einer bildlichen Aufnahme genutzt wird. Die Marker sind speziell entworfen, um einfach erkannt und identifiziert zu werden, und basieren auf einem binären Codierungssystem. Sie enthalten einzigartige, maschinenlesbare Muster, die es ermöglichen, ihre Position, Orientierung und Identität in einem Bild oder Video zu bestimmen. Jeder Marker trägt eine eindeutige ID, die durch die spezifische Anordnung der schwarzen und weißen Quadrate kodiert ist. Die Erkennung eines Aruco-Markers erfolgt typischerweise durch einen Algorithmus, der folgende Schritte durchführt: Das Bild scannt, um quadratische Konturen zu erkennen. Die Orientierung des Quadrats bestimmt. Den Code innerhalb des Quadrats entschlüsselt. Die ID des Markers sowie seine Position und Orientierung relativ zur Kamera berechnet.

Zur Generierung solcher Aruco-Marker ist z.B. die Aruco-Bibliothek in OpenCV (Open Source Computer Vision Library) zu verwenden. OpenCV dient ebenfalls zur Erkennung der Marker.

In einer weiteren bevorzugten Ausführungsform sind die ARUCO-Marker farblich umrandet (z.B. Pink und Türkis) zur besseren Erkennung auf verschiedenen Hauttönen.

Die ARUCO-Marker dienen als feste Referenzpunkte insbesondere zur Kalibrierung der Bildgröße und -perspektive. Vorzugsweise ist der Abstand zwischen zwei ARUCO-Markern festgelegt und dient als Skalierungshilfe und zur Berechnung der Pixelgröße des Bildes.

Daher betrifft die Erfindung eine Kalibrierung der bildlichen Aufnahme gemäß i.) mithilfe von ARUCO-Markern, die vorzugsweise farblich umrandet sind.

Besonders vorteilhaft erfolgt gemäß
ii.) die Bestimmung von mindestens einem Parameter ausgewählt aus der Gruppe Größe oder Volumen aus der bildlichen Aufnahme von mindestens zwei oder mehr Quaddeln aus i.) und / oder gemäß
iii.) Bestimmung der Verhältnisse der Parameter aus ii.) von mindestens zwei oder mehr Quaddeln,
   und/oder gemäß
iv.) wobei das Verhältnis der Allergene aus iii.) durch die Mengen an Allergenen in Gew. % repräsentiert ist, und die Mengen nach mindestens einem Parameter bestimmt werden ausgewählt aus der Gruppe Körpergewicht, Größe, Alter oder Geschlecht eines Patienten
   und/oder gemäß
v.) wobei das Verhältnis der Allergene aus iii.) durch die Mengen an Allergenen in Gew. % repräsentiert ist, und die Mengen nach mindestens einem Parameter bestimmt werden ausgewählt aus der Gruppe IgE-Level im Blut, Body Mass Index (BMI), saisonale Allergenbelastung, Vorerkrankungen jeweils mithilfe von Algorithmen.

Solche Algorithmen umfassen ebenfalls lernende Systeme oder Maschinen, insbesondere vorteilhaft zur Klassifizierung von Quaddeln, wie Entscheidungsbaum (Decision Tree): Basierend auf festen Grenzwerten für Größe, Volumen; Support Vector Machines (SVM): Zur Trennung der Klassen basierend auf mehrdimensionalen Daten; K-Means-Clustering: Unsupervised Learning zur Gruppierung der Quaddeln nach Ähnlichkeit; Convolutional Neural Network (CNN): Erweiterung des Deep-Learning-Modells zur Erkennung von komplexen Quaddelmustern und Formabweichungen, insbesondere zur Segmentierung der Quaddel anhand von Farbkontrasten und Rändern.

Besonders vorteilhaft können durch Algorithmen und lernende Systeme die Quaddeln insbesondere auch in der Zusammenschau mit den Parametern in iv.) und v.) klassifiziert werden.

Der Begriff Algorithmus bedeutet im weitesten Sinne dieser Erfindung eine endliche Abfolge von klar definierten Anweisungen oder Schritten, die dazu dienen, ein bestimmtes Problem zu lösen oder eine Aufgabe auszuführen, wie z.B. in der Informatik. Ein Algorithmus beschreibt einen systematischen Prozess, der in einer festgelegten Reihenfolge abgearbeitet wird, um ein gewünschtes Ergebnis zu erzielen.

Lernende Maschinen (Machine Learning (ML)) im Sinne dieser Erfindung sind Systeme oder Algorithmen, die aus Daten lernen können, anstatt explizit für jede Aufgabe programmiert zu werden. Sie nutzen statistische Methoden, um Muster in Daten zu erkennen und diese Erkenntnisse für Vorhersagen oder Entscheidungen zu verwenden.

### Funktionsweise lernender Maschinen:

Datenaufnahme: Die Maschine erhält eine große Menge von Daten, z. B. Zahlen, Texte, Bilder oder Videos.

Modelltraining: Ein Algorithmus wird mit diesen Daten trainiert, indem er Muster und Zusammenhänge erkennt.

Evaluation: Das trainierte Modell wird getestet, um sicherzustellen, dass es korrekte Ergebnisse liefert.

Anwendung: Das Modell wird auf neue, unbekannte Daten angewendet, um Vorhersagen oder Entscheidungen zu treffen.

Besonders vorteilhaft erfolgt gemäß ii.) die Bestimmung von mindestens einem Parameter ausgewählt aus der Gruppe Größe oder Volumen aus der bildlichen Aufnahme von mindestens zwei oder mehr Quaddeln aus i.) mithilfe von 3D Modellen.

Eine geeignete 3D Modellierung kann beispielsweise mithilfe Structure from Motion (SfM) und Multi-View Stereo (MVS) sowie den Bibliotheken OpenCV, OpenMVG, Open3D erfolgen.

Die Klassifikation der Quaddeln erfolgt vorteilhaft anhand mehrdimensionaler Daten (siehe unten, I. - VIII.), die über die Bildverarbeitung und ARUCO-Kalibrierung erfasst werden.

Mithilfe des Algorithmus oder lernende Maschine ist besonders vorteilhaft eine Klassifizierung der Quaddel neben Größe und Volumen anhand von mindestens einem Parameter ausgewählt aus der Gruppe:
I. Fläche der Quaddel (mm²): Automatische Berechnung der Pixelanzahl im Segmentierungsbereich.
II. Umfang (mm): Länge der Kontur der Quaddel.
III. Durchmesser (mm): Berechnung des maximalen Abstandes innerhalb der Kontur.
IV. Rötungsgrad (Rotwert im LAB-Farbraum): Durchschnittswert des Rotkanals.
V. Volumen (bei 3D-Rekonstruktion): Integration einer Höhenmessung zur Berechnung der Quaddeltiefe.
VI. Symmetrie: Analyse der Formregelmäßigkeit (Verhältnis von Länge zu Breite).
VII. Texturmerkmale: Überprüfung der Oberflächenbeschaffenheit (Homogenität, Kontrast, Entropie).
VIII. Erythema-Ausbreitung (Rötung): Ermittlung der roten Ausbreitungsfläche außerhalb der Quaddel.

Die pharmazeutischen Zusammensetzungen enthaltend Allergene, die je nach Applikation flüssig, teilweise flüssig oder fest sein können, und werden vorzugsweise in einer ersten festen Formulierung mithilfe eines 3D Druckers bereitgesellt, wobei die Allergene aus einer Bank entnommen werden, und mit geeigneten Hilfs- und Zusatzstoffen einem 3D Drucker zugeführt werden.

Die erforderlichen Mengen und Gewichtsverhältnisse in Gew. % der einzelnen Allergene können mittels Wiegen aus der Bank bereitgestellt werden.

Sofern erforderlich kann die erste feste Formulierung in eine flüssige oder teilweise flüssige Formulierung überführt werden.

Daher betrifft die Erfindung ein erfindungsgemäßes Verfahren zur Herstellung von pharmazeutischen Zusammensetzungen enthaltend Allergene, wobei die Mengen an Allergenen bzw. Mengenverhältnisse in Gew. % an Allergenen gemäß iii.) in einer festen Form mithilfe eines 3D Druckers eingebracht werden. Ein 3D-Drucker ist erfindungsgemäß ein Gerät, das digitale Modelle in physische, dreidimensionale Objekte umwandelt. Dabei wird Material, hier Allergen mit Zusatz- und Hilfsstoffen Schicht für Schicht aufgetragen bzw. eingebracht, bis das gewünschte Objekt, nämlich die pharmazeutische Zusammensetzung fertiggestellt ist.

Die pharmazeutische Zusammensetzung kann weitere Hilfs- und Zusatzstoffe enthalten, z.B. 5 bis 10 Gew. %, sodass sie mit 95 bis 90 Gew. % Allergene aud 100 Gew. % zu ergänzen ist.

Es sei darauf hingewiesen, dass Merkmale, die im Zusammenhang mit einer beispielhaften Ausführungsform oder einem beispielhaften Gegenstand beschrieben werden, mit jeder anderen beispielhaften Ausführungsform oder mit jedem anderen beispielhaften Gegenstand kombiniert werden können.

Wenn ein Begriff mit einem unbestimmten oder bestimmten Artikel, wie zum Beispiel "ein" im Singular bezeichnet wird, schließt dies auch den Begriff im Plural mit ein und umgekehrt, sofern der Kontext nicht eindeutig anderes festlegt.

Der Ausdruck "enthalten", wie er hier verwendet wird, schließt nicht nur die Bedeutung von "umfassen oder aufweisend" ein, sondern kann auch "bestehen(d) aus" und "im Wesentlichen bestehend aus" bedeuten.

Nachfolgende Beispiele dienen zur näheren Erläuterung der Erfindung, ohne jedoch die Erfindung auf diese Beispiele und Abbildungen zu begrenzen.

### Beispiele:

### Beispiel 1:

### Bildaufnahme und Kalibrierung mittels ARUCO-Marker

### Marker-Positionierung und Design

Zwei ARUCO-Marker werden auf der Haut angebracht.

Die Marker dienen als feste Referenzpunkte zur Kalibrierung der Bildgröße und -perspektive. Der Abstand zwischen den Markern ist festgelegt und dient als Skalierungshilfe.

### Technische Umsetzung:

Verwendung der OpenCV-Funktion cv2.aruco.detectMarkers() zur Erkennung der Marker. Berechnung des Abstands der Marker zueinander. Perspektivenkorrektur mittels cv2.getPerspectiveTransform().

### Kalibrierung des Bildmaßstabs

Die tatsächliche Pixelgröße des Bildes wird anhand des Abstands der ARUCO-Marker berechnet.

### Kamerawinkelkompensation

Der Barcode (ID) innerhalb der ARUCO-Marker ermöglicht die Berechnung des Neigungswinkels der Kamera.

Die Kameraneigung wird durch eine Homographie-Matrix kompensiert, um das Bild zu entzerren.

### Beispiel 2:

### Bildverarbeitungsschritte zur Quaddelerkennung

Nach der Kalibrierung erfolgt die Bildverarbeitung zur Erkennung der Quaddel.

Vorverarbeitung des Bildes:
Bildentzerrung: Korrektur perspektivischer Verzerrungen mittels Homographie.

Konvertierung in Graustufen: cv2.cvtColor() konvertiert das Bild für eine einfachere Verarbeitung.

Rauschreduktion: Gaussian Blur (cv2.GaussianBlur) zur Reduktion von Bildrauschen.

Segmentierung der Quaddel:
Ziel: Trennung der Quaddel vom restlichen Hautbild.

### Techniken:

Thresholding: cv2.threshold() zur binären Segmentierung basierend auf Helligkeit.

Kantendetektion: cv2.Canny() zur Kantenerkennung.

### Morphologische Operationen:

cv2.morphologyEx() zur Füllung kleiner Lücken und Reduktion von Artefakten.

### Merkmalsextraktion

Nach der Segmentierung werden folgende Merkmale der Quaddel extrahiert:

### Geometrische Merkmale:

Fläche (Area): Berechnung der Anzahl der Pixel innerhalb der Segmentierungsmaske:

Fläche=Σi,jI(i,j)\text{Fläche} = \sum_{i,j} I(i,j)Fla¨che=i,jz I(i,j)
(wobei I(i,j)I(i,j)I(i,j) der Pixelwert ist.)

Umfang (Perimeter): Berechnung mit der Funktion cv2.arcLength() basierend auf den Konturen.

Durchmesser: Maximaler Abstand zwischen zwei Punkten auf der Kontur.

### Farbmerkmale:

Mittlere Helligkeit: Durchschnitt der Pixelwerte innerhalb der Quaddel.

Rötungsgrad: Durchschnittswert im Rotkanal des LAB-Farbraums.

### Texturmerkmale:

Kantenstärke: Durchschnittliche Gradientenstärke der Quaddelränder.

Homogenität: Texturgleichmäßigkeit basierend auf dem GLCM (Gray-Level Co-occurrence Matrix).

### Beispiel 3:

### Allergologische Testergebnisse (Pricktest):

Milbenallergie: Quaddelgröße 15 mm²
Birkenpollenallergie: Quaddelgröße 13 mm²
Katzenhaarallergie: Quaddelgröße 9 mm²

Die Quaddelgröße ist ein direkter Indikator für die Schwere der allergischen Reaktion und bestimmt die empfohlene Dosis.

Größere Quaddeln zeigen eine stärkere Sensibilisierung und erfordern eine vorsichtigere Titration der Allergenmenge, um Überreaktionen zu vermeiden.

Die Klassifikation der Quaddelreaktionen ermöglicht eine standardisierte, objektive Basis für die Therapiedosierung.

Ohne ein datengestütztes Pricktestergebnis ist es nicht möglich, die individuelle Immunantwort des Patienten zu bewerten und eine personalisierte Tablette herzustellen. Die Quaddelmessung dient vorteilhaft als direkter Marker für die Immunreaktion und steuert somit sowohl die Therapiestartdosis als auch die Dynamik der Dosisanpassung während saisonaler Schwankungen.

## Patentansprüche

1. Verfahren zur Herstellung von pharmazeutischen Zusammensetzungen enthaltend Allergene aufweisend folgende Schritte:
i.) Bildliche Aufnahme von mindestens zwei oder mehr Quaddeln, welche durch einen Prick-Test erzeugt sind, wobei jede Quaddel mindestens ein verschiedenes Allergen repräsentiert,
ii.) Bestimmung von mindestens einem Parameter ausgewählt aus der Gruppe Größe oder Volumen aus der bildlichen Aufnahme von mindestens zwei oder mehr Quaddeln aus i.), iii.) Bestimmung der Verhältnisse der Parameter aus ii.) von mindestens zwei oder mehr Quaddeln,
**dadurch gekennzeichnet,**
**dass** die pharmazeutische Zusammensetzung entsprechend dem Verhältnis aus iii.) Allergene enthält.

2. Verfahren zur Herstellung von pharmazeutischen Zusammensetzungen enthaltend Allergene nach Anspruch 1 aufweisend folgenden weiteren Schritt:
iv.) wobei das Verhältnis der Allergene aus iii.) durch die Mengen an Allergenen in Gew. % repräsentiert ist, und die Mengen nach mindestens einem Parameter bestimmt werden ausgewählt aus der Gruppe Körpergewicht, Größe, Alter oder Geschlecht eines Patienten.

3. Verfahren zur Herstellung von pharmazeutischen Zusammensetzungen enthaltend Allergene nach Anspruch 1 aufweisend folgenden weiteren Schritt:
v.) wobei das Verhältnis der Allergene aus iii.) durch die Mengen an Allergenen in Gew. % repräsentiert ist, und die Mengen nach mindestens einem Parameter bestimmt werden ausgewählt aus der Gruppe IgE-Level im Blut, Body Mass Index (BMI), saisonale Allergenbelastung, Vorerkrankungen.

4. Verfahren zur Herstellung von pharmazeutischen Zusammensetzungen enthaltend Allergene nach einem der vorherigen Ansprüche zur Verwendung in der Immuntherapie oder in der Prophylaxe oder Behandlung von Allergien, Nahrungsmittelunverträglichkeit oder Arzneimittelunverträglichkeit.

5. Verfahren zur Herstellung von pharmazeutischen Zusammensetzungen enthaltend Allergene nach einem der vorherigen Ansprüche, wobei die Schritte ii.) und/oder iii.) und/oder iv.) und/oder v.) mithilfe von Algorithmen erfolgt.

6. Verfahren zur Herstellung von pharmazeutischen Zusammensetzungen enthaltend Allergene nach Anspruch 5, wobei die Algorithmen lernende Systeme oder Maschinen, Entscheidungsbaum (Decision Tree), Support Vector Machines (SVM), K-Means-Clustering, Convolutional Neural Network (CNN) umfassen.

7. Verfahren zur Herstellung von pharmazeutischen Zusammensetzungen enthaltend Allergene nach Anspruch 1, wobei eine Kalibrierung der bildlichen Aufnahme gemäß i.) mithilfe von ARUCO-Markern erfolgt, die vorzugsweise farblich umrandet sind.

8. Verfahren zur Herstellung von pharmazeutischen Zusammensetzungen enthaltend Allergene nach einem der vorherigen Ansprüche, wobei die Mengen an Allergenen in einer festen Form mithilfe eines 3D Druckers eingebracht werden.
